# EUROPEAN PATENT SPECIFICATION

(11) **EP 2 815 240 B1**
(45) Date of publication and mention of the grant of the patent: **27.07.2016**
(21) Application number: 13703603.4
(22) Date of filing: 12.02.2013
(51) Int. Cl.: G01N 33/68

(54) **USE OF THE SOLUBLE FORM OF AXL IN THE DIAGNOSIS AND/OR PROGNOSIS OF HEART FAILURE SYNDROME**
VERWENDUNG EINER LÖSLICHEN FORM VON AXL BEI DER DIAGNOSE UND/ODER PROGNOSE DES SYNDROMS DER HERZINSUFFIZIENZ
UTILISATION DE LA FORME SOLUBLE D'AXL POUR LE DIAGNOSTIC ET/OU LE PRONOSTIC DU SYNDROME D'INSUFFISANCE CARDIAQUE

(30) Priority: 13.02.2012 EP 12382048
(43) Date of publication of application: 24.12.2014
(73) Proprietor: Institut d'Investigacions Biomèdiques August Pi i Sunyer (IDIBAPS), 08036 Barcelona (ES); Consejo Superior De Investigaciones Científicas (CSIC), 28006 Madrid (ES)
(72) Inventor: BATLLE PERALES, Montserrat, 08031 Barcelona (ES); GARCÍA DE FRUTOS, Pablo, 08006 Barcelona (ES)
(74) Representative: Hoffmann Eitle
(86) International application number: PCT/EP2013/052743
(87) International publication number: WO 2013/120830

(56) References cited:
- WO-A1-2008/060871
- US-A- 5 468 634
- US-A1- 2003 157 573
- EKMAN C ET AL: "Plasma concentrations of Gas6 and soluble Axl correlate with disease and predict mortality in patients with critical limb ischemia", CLINICAL BIOCHEMISTRY, ELSEVIER INC, US, CA, vol. 43, no. 10-11, 1 July 2010 (2010-07-01), pages 873-876, XP027085691, ISSN: 0009-9120 [retrieved on 2010-06-13]

## Description

The present invention is generally related to diagnostic and prognostic assays. Particularly, the present invention refers to the identification and use of sAXL as a biomarker that is differentially expressed in patients suffering from heart failure syndrome.

### BACKGROUND ART

The Heart Failure (hereinafter also referred as "HF") syndrome can result from any structural or functional cardiac disorder that impairs the ability of the ventricle to fill with or eject blood. HF constitutes the endstage of many cardiopathies and is characterized by a ventricular dysfunction.

In 2008, the European Society of Cardiology (ESC) Guidelines for HF (Dickstein K et al., 2008) pointed out that the prevalence of HF is between 2-3% and rises sharply at 75 years of age, being the prevalence in 70-80 year old people between 10-20%. The overall prevalence of HF is increasing because of the ageing of the population, the success in prolonging survival in patients suffering coronary events, and the success in postponing coronary events by effective prevention. HF is the cause of 5% of acute hospital admissions, is the cause of 10% of patients in hospital beds and accounts for approximately 2% of national cost on health, mostly due to the cost of hospital admissions.

Two classifications of the severity of HF are employed (Dickstein K et al., 2008):
a) The New York Heart Association (NYHA) classification, which is based on symptoms and functional capacity. This classification has been proved to be clinically useful and it is used in most clinical trials. It classifies patients in four different classes:
   Class I, no limitation of physical activity. Ordinary physical activity does not cause undue fatigue, palpitation or dyspnoea.
   Class II, slight limitation of physical activity. Comfortable at rest, but ordinary physical activity results in fatigue, palpitation or dyspnoea.
   Class III, marked limitation of physical activity. Comfortable at rest, but less than ordinary physical activity results in fatigue, palpitation or dyspnoea.
   Class IV, unable to carry on any physical activity without discomfort. Symptoms at rest. If any physical activity is undertaken, discomfort is increased.
b) The American College of Cardiology/American Heart Association (hereinafter also referred as "ACC/AHA") (Jessup M. et al., 2009), which is based on the structural changes and symptoms of HF.

HF diagnosis is complex and requires a clinical examination, an electrocardiogram, a chest X-ray and an echocardiography. Several diagnostic tests are employed routinely to confirm HF but are more sensitive for the detection of patients with reduced ejection fraction than for patients with preserved ejection fraction (Dickstein K et al., 2008).

International patent application WO 2008/060871 A1 discloses the diagnosis of heart failure using tyrosine kinases as biomarker.

EKMAN C ET AL, CLINICAL BIOCHEMISTRY, ELSEVIER INC, US, CA, vol. 43, no. 10-11, 1 July 2010, pages 873-876 discloses that Axl and its ligand Gas6 are involved in and serve as biomarkers for critical limb ischemia. Furthermore, it is known from this document that Gas6 expression is involved in congestive heart failure.

In this regard, natriuretic peptides, B-type natriuretic peptide (BNP) and N-terminal pro-BNP (NT-proBNP) have been proposed as HF diagnostic tools, because elevated levels thereof lend support to a diagnosis of abnormal ventricular function or hemodynamics causing symptomatic HF. Elevated levels of natriuretic peptides should not be used in isolation to confirm or exclude the presence of HF. They may give weight to a suspected diagnosis of HF or trigger consideration of HF when the diagnosis is unknown (Jessup M. et al., 2004.

The main disadvantages for using natriuretic peptides as tools for HF diagnosis is their lability at room temperature and the cost of its measurement (Tang W. H. et al., 2009). Once the blood is retrieved from the patient, a cold cycle for preservation of BNP or NT-proBNP has to be established. Samples should be collected in iced tubes and processed rapidly to avoid *in vitro* degradation. These issues make the measurement of their levels a cumbersome process not available in many clinical settings. All these obstacles make it interesting to devise new methods to help diagnose and categorize HF.

In spite of the efforts made, therefore, there is still the need of further HF diagnostic tools.

### SUMMARY OF THE INVENTION

The inventors have revealed that the soluble form of AXL (hereinafter also referred as "sAXL") represents a newly realized biomarker particularly advantageous for predicting, diagnosing and/or prognosing heart failure (HF) syndrome.

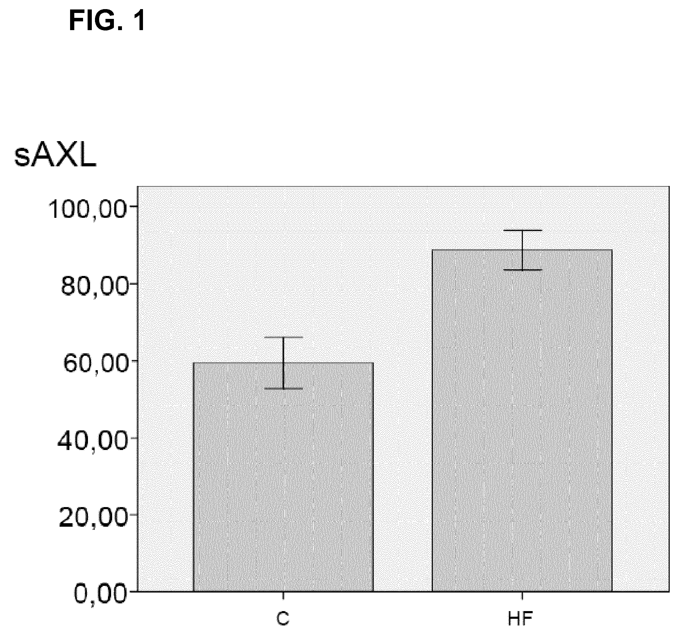

In particular, the present inventors have found that the soluble form of the AXL protein (hereinafter also referred as "sAXL") is increased in serum from HF patients compared to the levels detected in serum from subjects with no HF (see FIG. 1).

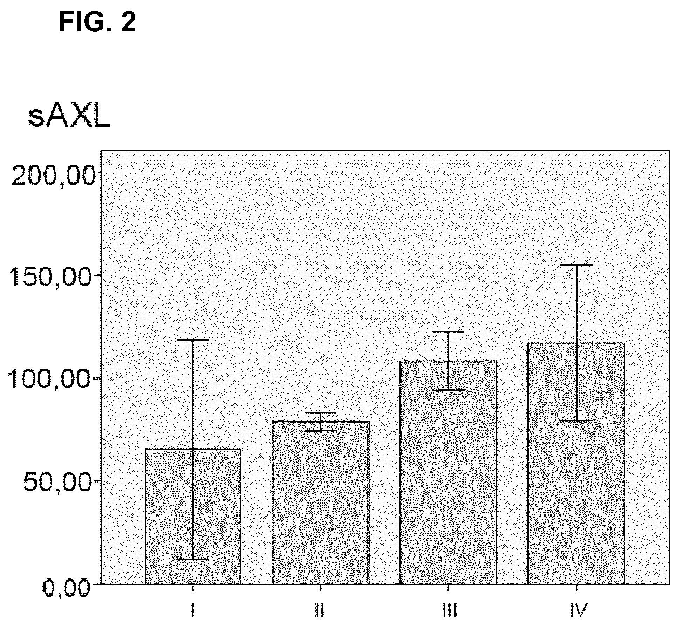

Furthermore, the inventors have found that the amount of sAXL is increased with NYHA functional classes, being the difference among functional classes statistically significant (see FIG. 2). This is indicative that sAXL provides useful prognosis information: higher sAXL level as more severe is the HF syndrome.

Thus, in a first aspect the present invention provides a method for diagnosing and/or prognosing a heart failure syndrome (HF), said method comprising the step of measuring, *in vitro,* the amount of the soluble form of AXL (sAXL) in a test sample.

As stated above, natriuretic peptides are used in the state of the art to determine HF syndrome. Said peptides are unstable and, if no adequate processing of the sample taken from the subject is made, the peptide is degraded, not being possible its detection. As a consequence of its degradation, the non-detection of said peptides in a test sample gives rise to false negative HF determinations, with the serious implications for the patient suffering the syndrome.

Surprisingly, and contrary to natriuretic peptides, sAXL is stable in serum. This confers two important advantages to the method of the first aspect of the invention when compared to the use of natriuretic peptides. The first one is that no cold preservation is needed for processing the sample; being the detection step of the method of the invention simpler. The second one is that using the method of the first aspect of the invention there can be a reduction of the false negative determinations associated with the use of natriuretic peptides as biomarkers, because no degradation of sAXL occurs.

Furthermore, it has been found that the difference in the sAXL expression profile between control subjects and subjects suffering from HF is so noticeable that a skilled doctor, with suitable means, would have a new tool to help in the diagnosis of the syndrome, with the resulting advantages for designing a suitable treatment.

In addition, the fact that the amount of sAXL measured in the test sample is compared with a control sample gives information about the prognosis of the syndrome: the higher the value of sAXL of a patient compared to the upper limit of sAXL reference range, the worse prognosis.

The method object of the first aspect of the invention can be useful in ruling out and ruling in HF in an acute setting with untreated patients; and stratifying the population, among others.

In addition, the method of the present invention is useful in diagnosing and/or prognosing HF associated with other previous cardiovascular diseases such as acute myocardial infarction or atrial fibrillation.

From the experimental data included in the present application, it can also be concluded that sAXL can be used as a marker for determining the risk for developing HF by performing the following steps:
a) determining, *in vitro,* the amount of sAXL in a test sample of the subject; being the subject a person with no heart failure syndrome but suspected that could develop it in the future; and
b) determining whether the sAXL amount determined in step (a) falls within a sAXL concentration reference range obtained from a group of subjects which do not have HF,
wherein, if the amount of sAXL in the test sample is close to the upper limit of the sAXL reference range, but without overcoming the upper value of said range, it is indicative that the patient has the risk of developing HF.

In a second aspect, the present invention provides a method of deciding or recommending whether to initiate a medical regimen of a subject suspected of suffering a heart failure syndrome, which method comprises the steps of:
a) determining, *in vitro,* the amount of sAXL in a test sample of the subject; and
b) diagnosing the heart failure syndrome if the sAXL amount in the test sample is higher than the upper limit of the sAXL concentration reference range obtained from a group of subjects which do not have HF, as defined in the method of the first aspect of the invention;
wherein:
i) if the subject is diagnosed of suffering a heart failure syndrome, then the initiation of the medical regimen is recommended, and
ii) if the patient is diagnosed of not suffering the heart failure syndrome, the follow-up of heart function is performed, optionally in consideration of the result of an examination of the patient by a cardiologist.

Determining the sAXL level in a test sample, the skilled person can establish, additionally, which is the most suitable therapy that can be recommended, because the level detected in the sample may reflect the extension (i.e., severity) of the syndrome.

Furthermore, once it has decided to initiate the medical regimen because the subject is suffering from HF syndrome (following the method defined in the second aspect of the invention), it can be monitored how efficient is the regimen: a decrease or return to a normal level of sAXL (i.e., to the level within the sAXL concentration reference range) can indicate that the HF patient has reacted favorably to the medical regimen and, therefore, said regimen is effective; if the level of sAXL does not significantly change or it increases, this can indicate that the medical regimen is not effective. Finally, the level of sAXL can be measured at the end of the treatment as part of the monitoring care for controlling relapses.

Therefore, in a third aspect, the present invention provides a method for determining the efficacy of a medical regimen in a patient already diagnosed of heart failure syndrome, the method comprising the steps of:
(a) *in vitro* measuring the amount of sAXL in a sample from the patient prior to initiating the medical regimen;
(b) *in vitro* measuring the amount of sAXL in a sample from the patient once started the medical regimen; and
(c) comparing the amounts measured in steps (a) and (b), in such a way that if the sAXL amount measured in step (b) is lower than the sAXL amount measured in step (a), it is indicative that the medical regimen is effective in the treatment of HF;
or, alternatively, the method comprising the steps of:
(i) *in vitro* measuring the amount of sAXL in a sample from the patient once initiated the medical regimen; and
(ii) determining whether the amount measured in step (i) falls within a sAXL concentration reference range obtained from a group of subjects which do not have HF,
wherein, if the sAXL amount measured in step (i) is within the sAXL concentration reference range, it is indicative that the medical regimen is effective in the treatment of HF.

In a fourth aspect, the present invention provides the use of sAXL as a marker for the diagnosis and/or prognosis of heart failure syndrome.

In a fifth aspect, the present invention provides the use of means for detecting the presence of sAXL in an isolated sample for the diagnosis and/or prognosis of heart failure syndrome in any of the methods defined above.

### BRIEF DESCRIPTION OF THE DRAWINGS

FIG. 1 is a bar representation showing the sAXL levels in serum from controls and HF patients. Particularly, each bar represents the mean sAXL concentration value and the error bars ± 2X standard error. Y-axis: sAXL= Mean sAXL level in serum (ng/ml); X-axis: C= serum from subjects that do not have HF selected for establishing the reference range, HF= serum from HF patients.
FIG. 2 is a bar representation showing the sAXL levels in serum from HF patients (n=149) belonging to NYHA I-IV functional classes. Particularly, each bar represents the mean sAXL concentration value and the error bars ± 2X standard error. Y-axis: sAXL= Mean sAXL level in serum (ng/ml); X-axis: 1= patients suffering from NYHA functional Class I, II= patients suffering from NYHA functional Class II, III= patients suffering from NYHA functional Class III, and IV= patients suffering from NYHA functional Class IV.

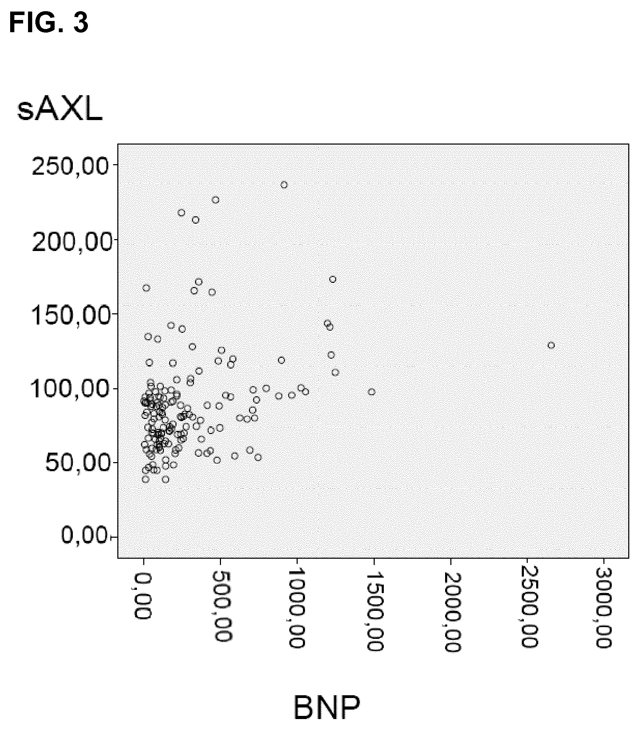

FIG. 3 shows the sAXL and BNP circulating levels in samples from HF patients, n=156. Y-axis: sAXL= sAXL level in serum (ng/ml); X-axis: BNP= BNP level in plasma (pg/ml).

### DETAILED DESCRIPTION OF THE INVENTION

As stated above, the present invention provides a method for diagnosing and prognosing a heart failure syndrome, said method being based on the measurement of the amount of sAXL.

AXL belongs to the receptor tyrosine kinases (RTKs) family. RTKs are membrane proteins recognizing extracellular signals leading to cellular responses as proliferation, arrest or activation. AXL sequence is available in several databases, such as NCBI databases (NP_068713.2) and Uniprot (database accession number: P30530; database version: 133).

In the present invention, the terms "soluble form of AXL" and "sAXL", have the same meaning and are interchangeable. The soluble form of AXL is the extracellular domain of AXL, which is located between the aminoacidic residues 26 to 451 of AXL protein sequence. Particularly, sAXL corresponds to SEQ ID NO: 1:
SEQ ID NO: 1
   APRGT QAEESPFVGN PGNITGARGL TGTLRCQLQV QGEPPEVHWL RDGQILELAD STQTQVPLGE DEQDDWIVVS QLRITSLQLS DTGQYQCLVF LGHQTFVSQP GYVGLEGLPY FLEEPEDRTV AANTPFNLSC QAQGPPEPVD LLWLQDAVPL ATAPGHGPQR SLHVPGLNKT SSFSCEAHNA KGVTTSRTAT ITVLPQQPRN LHLVSRQPTE LEVAWTPGLS GIYPLTHCTL QAVLSNDGMG IQAGEPDPPE EPLTSQASVP PHQLRLGSLH PHTPYHIRVA CTSSQGPSSW THWLPVETPE GVPLGPPENI SATRNGSQAF VHWQEPRAPL QGTLLGYRLA YQGQDTPEVL MDIGLRQEVT LELQGDGSVS NLTVCVAAYT AAGDGPWSLP VPLEAWRPGQ AQPVHQLVKE PSTPAFSWPW W

The term "heart failure" is to be understood as a syndrome in which the patients have the following features (Dickstein K et al., 2008): (a) symptoms such as shortness of breath at rest or during exertion, and/or fatigue; (b) signs of fluid retention such as pulmonary congestion or ankle swelling; and (c) objective evidence of an abnormality of the structure or function of the heart at rest.

The HF syndrome can result from any structural or functional cardiac disorder that impairs the ability of the ventricle to fill with or eject blood (Hunt S. A. et al., 2005) and constitutes the endstage of many cardiopathies and is characterised by a ventricular dysfunction. The most common causes of HF are acute or chronic ischaemia, hypertension, tachyarrhytmias, valvular syndrome and cardiomyopathies (such as hypertrophic, dilated, restrictive, arrhythmogenic right ventricular, myocarditis, among others). HF can also be induced by myocardial damage by drugs, toxins, nutritional or endocrine disorders and infiltrative cause such as sarcoidosis, amyloidosis, among others. Other causes of HF can be Chagas' disease, HIV infection, peripartum cardiomyopathy and end-stage renal failure (Dickstein K et al., 2008).

In the present invention the "sAXL concentration reference range obtained from a group of subjects which do not have HF" is to be understood as the control reference range in order to perform any of the methods object of the present invention. The subjects (controls) selected to determine the sAXL concentration reference range are chosen on the basis of the requirement that such subjects do not have HF. In order to determine the sAXL concentration range, it is determined the sAXL level in a sample taken from each subject (the sample being serum or plasma) and with the results obtained the 95% confidence limits of the reference group is statistically calculated.

The "test sample" refers to a sample obtained for the purpose of diagnosis and prognosis. In certain embodiments, such a sample may be obtained for the purpose of determining the outcome of an ongoing condition or to determine whether a medical regimen should be applied to a subject. Preferably the test sample is selected from the group consisting of: serum, plasma. More preferably, the test sample is serum.

The term "diagnosis" is known to the person skilled in the art. Diagnosing is understood as becoming aware of a particular medical condition, syndrome, complication or risk.

The term "subject" used herein and according to the present invention relates to a healthy individual, an apparently healthy individual, or an individual suffering from a syndrome (patient). The subject can be both a male and a female individual; particularly the patient is suffering from HF.

In the present invention, the expression "medical regimen" is to be understood as encompassing either pharmacological therapies (such as ACE-inhibitors, beta blockers, angiotensin receptor blockers, aldosterone antagonists, and digoxin, among others) as well as other clinical decisions taken by the cardiologist concerning, for instance, hospitalization or discharge decisions, or dietary or social habits pointed by the doctor such as salt ingestion or physical activity.

In the present invention, the expression "optionally in consideration of the result of an examination of the patient by a cardiologist" concerning the method of the second aspect of the invention, encompasses any clinical examination test useful in providing evidence of heart failure, such as electrocardiography, echocardiography, and chest X-ray, among others.

In a preferred embodiment of the first aspect, the method comprises the steps of:
(a) measuring, *in vitro,* the amount of sAXL in the test sample; and
(b) determining whether the amount determined in step (a) falls within a sAXL concentration reference range obtained from a group of subjects
which do not have HF,
wherein, if the amount of sAXL in the test sample is higher than the upper limit of the sAXL reference range, it is indicative that the patient is suffering or has a bad prognosis of HF syndrome.

In a preferred embodiment of the method of the first, second, and third aspects of the invention, the amount of sAXL protein present in the sample is determined.

In order to carry out the protein detection, an antibody or a fragment thereof with the ability of binding to sAXL can be used.

In the present invention, the term "antibody or a fragment thereof with the ability of binding to sAXL" is to be understood as any immunoglobulin or fragment thereof able to bind the antigen defined by sAXL. It includes monoclonal and polyclonal antibodies. The term "fragment thereof" encompasses any part of an antibody having the size and conformation suitable to bind an epitope of sAXL. Suitable fragments include F(ab), F(ab') and Fv. An "epitope" is the part of the antigen being recognized by the immune system (B-cells, T-cells or antibodies). Suitable antibodies may be those mentioned in the example below, such as AF154 (R&D systems), which is derived from a mouse myeloma cell line NS0 derived recombinant human AXL Glu33Pro440 (Accession # AAA61243); and PAB2998 (Abnova).

In a preferred embodiment, the antibody or fragment thereof for detecting sAXL is included in a kit. The kit may additionally comprise means (additives, solvents) to visualize the antigen-antibody interactions (dipsticks, chemiluminescent reagents, turbidimetric reagents, etc.). Suitable additives, solvents and reagents to visualize the antigen-antibody interaction are disclosed in the examples.

In another preferred embodiment, the methods of the first, second and third aspect of the invention, are competitive enzyme linked immunosorbent assay (ELISA) methods. In this embodiment, the *in vitro* measuring of sAXL amount (either in the group of subjects used for determining the sAXL concentration reference range or the test sample) is performed using a sAXL capture antibody, preferably a sAXL polyclonal capture antibody.

Throughout the description and claims the word "comprise" and variations of the word, are not intended to exclude other technical features, additives, components, or steps. Furthermore, the word "comprise" and its variations encompasses the term "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### 1. Patient enrolment and collection of clinical data

This was a prospective study of HF patients with ischemic, idiopathic, valvular and other ethiologies who belong to New York Heart Association (NYHA) functional classes I, II, III or IV. These patients underwent a history and echocardiography examination. Clinical data such as HF evolution time, number of meters walked in the 6 minute walking test, cardiovascular risk factors (hypertension, dyslipidaemia, diabetes mellitus and smoking), and medication were entered for analysis, along with ventricular dilation parameters measured by echocardiography, such as left ventricle end-systolic diameter (LVESD), left ventricle end-dyastolic diameter (LVESD) and EF ("ejection fraction") as a parameter of systolic function.

The Ethical Committee of the affiliated institution approved the protocol and all subjects gave written, informed consent to participate in this study.

Patients were excluded if they were affected by other fibroproliferative diseases such as; kidney failure, cirrhosis, bone metabolic disease, hyperthyroidism, pulmonary fibrosis, systemic sclerosis and macular degeneration.

All subjects selected for determining the sAXL reference range (hereinafter also referred "control subjects") have no heart failure. A subgroup of these subjects did not present any heart failure symptoms and another subgroup of these subjects were clinically evaluated by a cardiologist to rule out HF.

### 2. Collection of serum samples

Blood samples were collected from an antecubital vein from 188 HF patients and from 66 controls.

Serum samples for sAXL analysis were kept at room temperature for at least 30 minutes after blood extraction, to let the clot to be formed and were later centrifuged at a relative centrifugal force (RCF) 1800 x g for 10 minutes at room temperature. The supernatant was collected, aliquoted and kept at -80 °C until analysis.

To measure circulating levels of Brain Natriuretic Peptide (BNP) whole blood was collected in a chilled tube with the anticoagulant EDTA. The sample was promptly put on ice after blood extraction and was centrifuged at RCF 1800 x g for 10 minutes at 4 °C. The supernatant was collected, aliquoted and kept at -80 °C until analysis.

### 3. Collection and data analysis

Statistics were calculated with the SPSS software, version 18.0 (SPSS Institute Inc, Cary, NC, USA). The non-parametric Mann-Whitney test was applied to compare the sAXL values between controls and HF patients. The non-parametric Kruskal Wallis test was applied to analyze for sAXL differences among functional classes in HF patients. The Spearman correlation coefficient was used to analyse for putative association among the circulating sAXL and BNP levels. Numerical values in the text are mean sAXL values ± standard error of the mean. In all cases, a p value<0.05 was considered to be statistically significant.

In this study an ELISA method to quantify soluble AXL levels in serum was designed. For the determination of sAXL, the polyclonal antibody AF154 (R&D systems) was used as capture antibody, and the biotinylated BAF154 (R&D Systems) was used as detection antibody.

Multiwell plates (96 wells, Rubilabor) were coated with the capture antibody AF154 at a concentration of 2.0 µg/mL in phosphate buffered saline (PBS) (137 mM NaCl, 2.7 mM KCI, 8.1 mM Na₂HPO₄, 1.5 mM KH₂PO₄, pH 7.2, filtered through a 0.2 µm pore). The plate is sealed and left overnight at room temperature. The plates were washed three times with wash buffer (PBS with 0,05% Tween20) and then plates were blocked for 1 hour at room temperature with PBS containing 1 % BSA (Sigma).

Serum samples were diluted 200 times in PBS containing 1% bovine serum albumin (BSA). A standard curve was made by serial dilution of a purified sAXL protein produced by recombinant expression of the extracellular part of AXL obtained from R&D systems (154-AL-100). A negative control without serum sample was used and pooled-plasma samples were used in each plate. After sample addition, the plates were washed three times with wash buffer (PBS with 0,05% Tween20). Then, 100 µL of the BAF154, diluted in PBS containing 1 % BSA was added at a concentration of 50 ng/mL and incubated 2 hours at room temperature. The aspiration/wash steps were repeated and 100 µL of the working dilution of Streptavidin-HRP (Sigma) were added to each well. Cover the plate and incubate for 20 minutes at room temperature. The aspiration/wash steps are repeated and a solution of tetramethyl-benzidine (slow kinetic form; Sigma) was added to the well. The reaction was stopped with sulfuric acid at 1 M concentration. The absorbance of wells was determined with an automatic plate reader at 450 nm.

### 4. Results:

In FIG. 1 it is shown that sAXL levels in serum from HF patients is statistically higher (88.5±2.55 ng/ml, n=188) than in serum from control subjects (59.4±3.4 ng/ml, n=66, p<0.001*, Mann-Whitney test). This is indicative that sAXL is a good diagnostic marker of HF syndrome.

In FIG. 2 it is shown the mean sAXL values ± 2x standard error of the mean for each one of the four classes distinguished by NYHA:
Class I: 65.4±26.7 ng/ml (n=2);
Class II: 78.7±2.2 ng/ml (n=98),
Class III: 108.4±7.1 ng/ml (n=45), and
Class IV: 117.2±19 ng/ml (n=5).

From the results obtained, it can be concluded that sAXL levels increase with NYHA functional classes and, therefore, with the severity of the syndrome. The difference among functional classes is statistically significant (p<0.001*, Kruskal Wallis test).

Therefore, it can be concluded that sAXL is a prognosis marker of HF: the higher the value of sAXL of a patient compared to sAXL levels in controls, the worse the prognosis.

In FIG. 3 it is shown a significant correlation between sAXL and BNP circulating levels in HF patients (n= 156, p<0.001, R= 0.304, Spearman Test).

sAXL levels correlate with BNP levels, the current marker of HF. Due to this correlation, it can be concluded that sAXL is a marker of diagnosis and prognosis of HF, providing technical advantages to BNP measurements due to its stability in serum (in contrast to BNP, which is unstable).

### REFERENCES CITED IN THE APPLICATION

Dickstein K et al., "ESC Guidelines for the diagnosis and treatment of acute and chronic heart failure 2008: the Task Force for the Diagnosis and Treatment of Acute and Chronic Heart Failure 2008 of the European Society of Cardiology. Developed in collaboration with the Heart Failure Association of the ESC (HFA) and endorsed by the European Society of Intensive Care Medicine (ESICM)", 2008, Eur.Heart J., vol. 29, p. 2388-2442;

Hunt S. A. et al.," ACC/AHA 2005 Guideline Update for the Diagnosis and Pattern of Chronic Heart Failure in the Adult: a report of the American College of Cardiology/American Heart Association Task Force on Practice Guidelines (Writing Committee to Update the 2001 Guidelines for the Evaluation and Pattern of Heart Failure): developed in collaboration with the American College of Chest Physicians and the International Society for Heart and Lung Transplantation: endorsed by the Heart Rhythm Society", 2005, Circulation; vol. 112, p. e154-e235.

Jessup M. et al., "2009 focused update: ACCF/AHA Guidelines for the Diagnosis and Pattern of Heart Failure in Adults: a report of the American College of Cardiology Foundation/American Heart Association Task Force on Practice Guidelines: developed in collaboration with the International Society for Heart and Lung Transplantation", 2009, Circulation, vol. 119, p. 1977-2016.

Tang W. H. et al., "National Academy of Clinical Biochemistry Laboratory Medicine practice guidelines: Clinical utilization of cardiac biomarker testing in heart failure", 2007, Circulation; vol. 116, p. e99-109.

Wong M. et al., "Severity of left ventricular remodeling defines outcomes and response to therapy in heart failure: Valsartan heart failure trial (Val-HeFT) echocardiographic data", 2004, J.Am.Coll.Cardiol.;vol. 43, p. 2022-2027.

Wu A. H. et al., "Biological variation for N-terminal pro- and B-type natriuretic peptides and implications for therapeutic monitoring of patients with congestive heart failure", 2003, Am. J. Cardiol., vol. 92, p. 628-631.

WO 2008/060871 A1 (MAYO FOUNDATION [US]; BROZOVICH FRANK V [US]; OGUT OZGUR [US]; CHEN FR) 22 May 2008 (2008-05-22).

EKMAN C ET AL: "Plasma concentrations of Gas6 and soluble Axl correlate with disease and predict mortality in patients with critical limb ischemia",CLINICAL BIOCHEMISTRY, ELSEVIER INC, US, CA, vol. 43, no. 10-11, 1 July 2010 (2010-07-01), pages 873-876.

## Claims

1. A method for diagnosing and/or prognosing a heart failure syndrome (HF), said method comprising the step of measuring, *in vitro,* the amount of the soluble form of AXL (sAXL) in a test sample.

2. The method of claim 1 comprising the steps of
(a) measuring, *in vitro,* the amount of sAXL in the test sample; and
(b) determining whether the amount determined in step (a) falls within a sAXL concentration reference range obtained from a group of subjects which do not have HF,
wherein, if the amount of sAXL in the test sample is higher than the upper limit of the sAXL reference range, it is indicative that the patient is suffering or has a bad prognosis of HF syndrome.

3. The method of any of the preceding claims, wherein it is determined the protein amount of sAXL in the test sample.

4. The method of any of the preceding claims, wherein the test sample is selected from the group consisting of: serum, and plasma.

5. A method of deciding or recommending whether to initiate a medical regimen of a subject suspected of suffering a heart failure syndrome, which method comprises the steps of:
a) determining, *in vitro,* the amount of sAXL in a test sample of the subject; and
b) diagnosing the heart failure syndrome if the sAXL is higher than the upper limit of the sAXL concentration reference range obtained from a group of subjects which do not have HF;
wherein:
i) if the subject is diagnosed of suffering a heart failure syndrome, then the initiation of the medical regimen is recommended, and
ii) if the patient is diagnosed of not suffering the heart failure syndrome, the follow-up of heart function is performed optionally in consideration of the result of an examination of the patient by a cardiologist.

6. A method for determining the efficacy of a medical regimen in a patient already diagnosed of heart failure syndrome, the method comprising the steps of:
(a) *in vitro* measuring the amount of sAXL in a sample from the patient prior to the medical regimen:
(b) *in vitro* measuring the amount of sAXL in a sample from the patient once started the medical regimen; and
(c) comparing the amounts measured in steps (a) and (b), in such a way that if the sAXL amount measured in step (b) is lower than the sAXL amount measured in step (a), it is indicative that the medical regimen is effective in the treatment of HF;
or, alternatively, the method comprising the steps of:
(i) *in vitro* measuring the amount of sAXL in a sample from the patient once started the medical regimen; and
(ii) determining whether the amount measured in step (i) falls within a sAXL concentration reference range obtained from a group of subjects which do not have HF,
wherein, if the sAXL amount measured in step (i) is within the sAXL concentration reference range, it is indicative that the medical regimen is effective in the treatment of HF.

7. The methods of any of claims 5-6, wherein it is determined the protein amount of sAXL in the test sample.

8. The methods of any of claims 5-7, wherein the test sample is selected from the group consisting of: serum and plasma.

9. The method of any of claims 1-8, which is a competitive enzyme linked immunosorbent assay (ELISA) method.

10. The method of claim 9, wherein the *in vitro* measuring of sAXL amount is performed using a sAXL capture antibody.

11. Use of sAXL as a marker for the diagnosis and/or prognosis of heart failure syndrome.

12. Use of means for detecting the presence of sAXL in an isolated sample for the diagnosis and/or prognosis of heart failure syndrome in any of the methods of claims 1 to 10.

13. The use of claim 12, wherein the means is an antibody or a fragment thereof which specifically binds to sAXL.

14. The use of claim 13, wherein the antibody or fragment thereof, forms part of a kit.

## Patentansprüche

1. Verfahren für die Diagnose und/oder Prognose eines Syndroms der Herzinsuffizienz (HF), wobei das genannte Verfahren den Schritt der Messung, *in vitro,* der Menge der löslichen Form von AXL (sAXL) in einer Testprobe, umfasst.

2. Verfahren nach Anspruch 1, umfassend die Schritte
(a) der Messung, *in vitro,* der Menge von sAXL in der Testprobe; und
(b) der Bestimmung ob die in Schritt (a) bestimmte Menge innerhalb eines Referenzbereiches der sAXL-Konzentration liegt, welcher aus der Gruppe von Probanden die keine HF aufweisen erhalten wird,
wobei, wenn die Menge von sAXL in der Testprobe höher als die obere Grenze des Referenzbereiches der sAXL ist, dies dafür indikativ ist, dass der Patient unter Syndrom der HF leidet oder eine schlechte Prognose von Syndrom der HF aufweist.

3. Verfahren nach einem der vorhergehenden Ansprüche, wobei die Proteinmenge von sAXL in der Testprobe bestimmt wird.

4. Verfahren nach einem der vorhergehenden Ansprüche, wobei die Testprobe aus der Gruppe ausgewählt wird, bestehend aus: Serum und Plasma.

5. Verfahren zum Entscheiden oder Empfehlen ob mit einer medizinischen Kur eines Probanden, von dem vermutet wird, das er unter einem Syndrom der Herzinsuffizienz leidet, begonnen werden soll, welches Verfahren die Schritte:
a) der Bestimmung, *in vitro,* der Menge von sAXL in einer Testprobe des Probanden; und
b) des Diagnostizierens des Syndroms der Herzinsuffizienz wenn die sAXL höher als die obere Grenze des Referenzbereiches der sAXL-Konzentration ist, welcher aus der Gruppe von Probanden die keine HF aufweisen erhalten wird;
umfasst,
wobei:
i) wenn der Proband diagnostiziert wird, dass er unter einem Syndrom der Herzinsuffizienz leidet, dann der Beginn der medizinischen Kur empfohlen wird, und
ii) wenn der Patient diagnostiziert wird, dass er nicht unter dem Syndrom der Herzinsuffizienz leidet, die Überwachung der Herzfunktion gegebenenfalls durchgeführt wird, unter Berücksichtigung des Ergebnisses einer Untersuchung des Patienten durch einen Kardiologen.

6. Verfahren zum Bestimmen der Wirksamkeit einer medizinischen Kur in einem bereits mit Syndrom der Herzinsuffizienz diagnostizierten Patient, wobei das Verfahren die Schritte:
(a) der *in vitro*-Messung der Menge von sAXL in einer Probe des Patienten vor der medizinischen Kur;
(b) der *in vitro*-Messung der Menge von sAXL in einer Probe des Patienten sobald mit der medizinischen Kur begonnen wurde; und
(c) des Vergleichens der in den Schritten (a) und (b) gemessenen Mengen, sodass, wenn die in Schritt (b) gemessene sAXL-Menge niedriger als die in Schritt (a) gemessene sAXL-Menge ist, dies dafür indikativ ist, dass die medizinische Kur wirksam bei der Behandlung von HF ist;
umfasst,
oder, alternativ, wobei das Verfahren die Schritte:
(i) der *in vitro*-Messung der Menge von sAXL in einer Probe des Patienten sobald mit der medizinischen Kur begonnen wurde; und
(ii) der Bestimmung ob die in Schritt (i) gemessene Menge innerhalb eines Referenzbereiches der sAXL-Konzentration liegt, welcher aus einer Gruppe von Probanden die keine HF aufweisen erhalten wird,
umfasst,
wobei, wenn die in Schritt (i) gemessene sAXL-Menge innerhalb des Referenzbereiches der sAXL-Konzentration ist, dies dafür indikativ ist, dass die medizinische Kur wirksam bei der Behandlung von HF ist.

7. Verfahren nach einem der Ansprüche 5-6, wobei die Proteinmenge von sAXL in der Testprobe bestimmt wird.

8. Verfahren nach einem der Ansprüche 5-7, wobei die Testprobe aus der Gruppe ausgewählt wird, bestehend aus: Serum und Plasma.

9. Verfahren nach einem der Ansprüche 1-8, welches ein konkurrierendes enzymgekoppeltes Immunadsorptionstest (ELISA)-Verfahren ist.

10. Verfahren nach Anspruch 9, wobei das *in vitro*-Messen der sAXL-Menge unter Verwendung eines sAXL-Fängerantikörpers durchgeführt wird.

11. Verwendung von sAXL als Marker für die Diagnose und/oder Prognose eines Syndroms der Herzinsuffizienz.

12. Verwendung von Mitteln zur Ermittlung der Anwesenheit von sAXL in einer isolierten Probe für die Diagnose und/oder Prognose eines Syndroms der Herzinsuffizienz in einem der Verfahren nach den Ansprüchen 1 bis 10.

13. Verwendung nach Anspruch 12, wobei das Mittel ein Antikörper oder ein Fragment desselben ist, dass spezifisch die sAXL bindet.

14. Verwendung nach Anspruch 13, wobei der Antikörper oder Fragment desselben, Teil eines Kits ist.

## Revendications

1. Méthode de diagnostic et/ou pronostic d'un syndrome d'insuffisance cardiaque (HF), ladite méthode comprenant l'étape consistant à mesurer, *in vitro,* la quantité de la forme soluble d'AXL (sAXL) dans un échantillon d'essai.

2. Méthode selon la revendication 1 comprenant les étapes consistant à
(a) mesurer, *in vitro,* la quantité de sAXL dans l'échantillon d'essai ; et
(b) déterminer si la quantité déterminée à l'étape (a) se situe dans une plage de référence de concentration de sAXL obtenue à partir d'un groupe de sujets qui n'ont pas de HF,
où, si la quantité de sAXL dans l'échantillon d'essai est supérieure à la limite supérieure de la plage de référence de sAXL, cela indique que le patient souffre ou a un mauvais pronostic du syndrome de HF.

3. Méthode selon l'une quelconque des revendications précédentes, où la quantité de protéine sAXL dans l'échantillon d'essai est déterminée.

4. Méthode selon l'une quelconque des revendications précédentes, où l'échantillon d'essai est choisi parmi le groupe constitué de : sérum et plasma.

5. Méthode pour décider ou recommander d'initier ou non un traitement médical d'un sujet soupçonné de souffrir d'un syndrome d'insuffisance cardiaque, ladite méthode comprenant les étapes consistant à :
a) déterminer, *in vitro,* la quantité de sAXL dans un échantillon d'essai du sujet ; et
b) diagnostiquer le syndrome d'insuffisance cardiaque si le sAXL est supérieure à la limite supérieure de la plage de référence de concentration de sAXL obtenue à partir d'un groupe de sujets qui n'ont pas de HF ;
où :
i) s'il est diagnostiqué que le sujet souffre d'un syndrome d'insuffisance cardiaque, alors l'initiation du traitement médical est recommandée, et
ii) s'il est diagnostiqué que le patient ne souffre pas du syndrome d'insuffisance cardiaque, le suivi de la fonction cardiaque est réalisé de façon optionnelle en fonction du résultat d'un examen du patient par un cardiologue.

6. Méthode pour déterminer l'efficacité d'un traitement médical chez un patient déjà diagnostiqué d'un syndrome d'insuffisance cardiaque, la méthode comprenant les étapes consistant à :
(a) mesurer *in vitro* la quantité de sAXL dans un échantillon provenant du patient avant le traitement médical ;
(b) mesurer *in vitro* la quantité de sAXL dans un échantillon provenant du patient une fois le traitement médical commencé ; et
(c) comparer les quantités mesurées aux étapes (a) et (b), de telle manière que si la quantité de sAXL mesurée à l'étape (b) est inférieure à la quantité de sAXL mesurée à l'étape (a), cela indique que le traitement médical est efficace dans le traitement de HF;
ou, alternativement, la méthode comprenant les étapes consistant à :
(i) mesurer *in vitro* la quantité de sAXL dans un échantillon provenant du patient une fois le traitement médical commencé ; et
(ii) déterminer si la quantité mesurée à l'étape (i) se situe dans une plage de référence de concentration de sAXL obtenue à partir d'un groupe de sujets qui n'ont pas de HF,
où, si la quantité de sAXL mesurée à l'étape (i) se situe dans la plage de référence de concentration de sAXL, cela indique que le traitement médical est efficace dans le traitement de HF.

7. Méthodes selon l'une quelconque des revendications 5-6, où la quantité de protéine sAXL dans l'échantillon d'essai est déterminée.

8. Méthodes selon l'une quelconque des revendications 5-7, où l'échantillon d'essai est choisi parmi le groupe constitué de : sérum et plasma.

9. Méthode selon l'une quelconque des revendications 1-8, qui est une méthode de dosage d'immunoadsorption par enzyme liée (ELISA) par compétition.

10. Méthode selon la revendication 9, où la mesure *in vitro* de la quantité de sAXL est réalisée en utilisant un anticorps de capture de sAXL.

11. Utilisation de sAXL comme un marqueur pour le diagnostic et/ou le pronostic du syndrome d'insuffisance cardiaque.

12. Utilisation d'un moyen pour détecter la présence de sAXL dans un échantillon isolé pour le diagnostic et/ou le pronostic du syndrome d'insuffisance cardiaque dans l'une quelconque des méthodes selon les revendications 1 à 10.

13. Utilisation selon la revendication 12, où le moyen est un anticorps ou un fragment de celui-ci qui se lie spécifiquement au sAXL.

14. Utilisation selon la revendication 13, où l'anticorps ou le fragment de celui-ci fait partie d'un kit.


### SEQUENCE LISTING

<110> Institut d'Investigacions Biomèdiques August Pi i Sunyer Consejo Superior de Investigaciones Cientificas
<120> Use of the soluble form of AXL in the diagnosis and/or prognosis of heart failure syndrome
<130> P2159PC00
<150> EP 12382048
   <151> 2012-02-13
<160> 1
<170> PatentIn version 3.5
<210> 1
   <211> 426
   <212> PRT
   <213> Homo sapiens
<400> 1
   Ala Pro Arg Gly Thr Gln Ala Glu Glu Ser Pro Phe Val Gly Asn Pro
   1               5                   10                  15
   Gly Asn Ile Thr Gly Ala Arg Gly Leu Thr Gly Thr Leu Arg Cys Gln
               20                  25                  30
   Leu Gln Val Gln Gly Glu Pro Pro Glu Val His Trp Leu Arg Asp Gly
           35                  40                  45
   Gln Ile Leu Glu Leu Ala Asp Ser Thr Gln Thr Gln Val Pro Leu Gly
       50                  55                  60
   Glu Asp Glu Gln Asp Asp Trp Ile Val Val Ser Gln Leu Arg Ile Thr
   65                  70                  75                  80
   Ser Leu Gln Leu Ser Asp Thr Gly Gln Tyr Gln Cys Leu Val Phe Leu
                   85                  90                  95
   Gly His Gln Thr Phe Val Ser Gln Pro Gly Tyr Val Gly Leu Glu Gly
               100                 105                 110
   Leu Pro Tyr Phe Leu Glu Glu Pro Glu Asp Arg Thr Val Ala Ala Asn
           115                 120                 125
   Thr Pro Phe Asn Leu Ser Cys Gln Ala Gln Gly Pro Pro Glu Pro Val
       130                 135                 140
   Asp Leu Leu Trp Leu Gln Asp Ala Val Pro Leu Ala Thr Ala Pro Gly
   145                 150                 155                 160
   His Gly Pro Gln Arg Ser Leu His Val Pro Gly Leu Asn Lys Thr Ser
                   165                 170                 175
   Ser Phe Ser Cys Glu Ala His Asn Ala Lys Gly Val Thr Thr Ser Arg
               180                 185                 190
   Thr Ala Thr Ile Thr Val Leu Pro Gln Gln Pro Arg Asn Leu His Leu
           195                 200                 205
   Val Ser Arg Gln Pro Thr Glu Leu Glu Val Ala Trp Thr Pro Gly Leu
       210                 215                 220
   Ser Gly Ile Tyr Pro Leu Thr His Cys Thr Leu Gln Ala Val Leu Ser
   225                 230                 235                 240
   Asn Asp Gly Met Gly Ile Gln Ala Gly Glu Pro Asp Pro Pro Glu Glu
                   245                 250                 255
   Pro Leu Thr Ser Gln Ala Ser Val Pro Pro His Gln Leu Arg Leu Gly
               260                 265                 270
   Ser Leu His Pro His Thr Pro Tyr His Ile Arg Val Ala Cys Thr Ser
           275                 280                 285
   Ser Gln Gly Pro Ser Ser Trp Thr His Trp Leu Pro Val Glu Thr Pro
       290                 295                 300
   Glu Gly Val Pro Leu Gly Pro Pro Glu Asn Ile Ser Ala Thr Arg Asn
   305                 310                 315                 320
   Gly Ser Gln Ala Phe Val His Trp Gln Glu Pro Arg Ala Pro Leu Gln
                   325                 330                 335
   Gly Thr Leu Leu Gly Tyr Arg Leu Ala Tyr Gln Gly Gln Asp Thr Pro
               340                 345                 350
   Glu Val Leu Met Asp Ile Gly Leu Arg Gln Glu Val Thr Leu Glu Leu
           355                 360                 365
   Gln Gly Asp Gly Ser Val Ser Asn Leu Thr Val Cys Val Ala Ala Tyr
       370                 375                 380
   Thr Ala Ala Gly Asp Gly Pro Trp Ser Leu Pro Val Pro Leu Glu Ala
   385                 390                 395                 400
   Trp Arg Pro Gly Gln Ala Gln Pro Val His Gln Leu Val Lys Glu Pro
                   405                 410                 415
   Ser Thr Pro Ala Phe Ser Trp Pro Trp Trp
               420                 425